# EUROPÄISCHE PATENTSCHRIFT

(11) **EP 2 384 706 B1**
(45) Veröffentlichungstag und Bekanntmachung des Hinweises auf die Patenterteilung: **11.11.2015**
(21) Anmeldenummer: 11164376.3
(22) Anmeldetag: 29.04.2011
(51) Int. Cl.: A61B 17/16

(54) **Chirurgisches Instrument und chirurgisches Instrumentarium**
Surgical instrument and set of surgical instruments
Instrument chirurgical et instrumentation chirurgicale

(30) Priorität: 05.05.2010 DE 102010016802
(43) Veröffentlichungstag der Anmeldung: 09.11.2011
(73) Patentinhaber: Aesculap AG, 78532 Tuttlingen (DE)
(72) Erfinder: Fritzsche, JÀ¶rg, 78665 Frittlingen (DE)
(74) Vertreter: Hoeger, Stellrecht & Partner Patentanwälte mbB

(56) Entgegenhaltungen:
- WO-A1-02/49516
- DE-U- 7 113 620
- GB-A- 2 397 768
- US-A1- 2003 078 587

## Beschreibung

Die Erfindung betrifft ein chirurgisches Instrument zur Erzeugung einer halbkugelförmigen Ausnehmung in einem Knochen, das um eine Drehachse drehend antreibbar ist und mindestens ein distales Schneidelement mit einer Schneidkante zur Abtragung von Knochenmaterial umfasst, wobei die Schneidkante in Rotation des Instrumentes um die Drehachse eine Bearbeitungssphäre mit einer in die Ausnehmung einführbaren distalen Hemisphäre, einem Äquator und einer proximalen Hemisphäre definiert, wobei das Instrument einen als Hohlkörper ausgebildeten Grundkörper umfasst, an dem die Schneidelemente festgelegt sind, und wobei das Instrument mindestens ein proximales Schneidelement mit einer Schneidkante umfasst, die sich ausgehend von der Äquatorialebene der Bearbeitungssphäre oder im Wesentlichen von der Äquatorialebene in proximaler Richtung erstreckt.

Außerdem betrifft die Erfindung ein chirurgisches Instrumentarium.

"Proximal" und "distal" sind vorliegend auf den das Instrument bedienenden Operateur bezogen aufzufassen. Dieser bedient das Instrument auf dessen proximaler Seite und wirkt mit dessen distaler Seite auf den Knochen ein.

Als "distales Schneidelement" wird vorliegend ein Schneidelement angesehen, dessen Schneidkante einen auf der distalen Hemisphäre der Bearbeitungssphäre angeordneten Abschnitt aufweist.

Ein Instrument der eingangs genannten Art wird beispielsweise bei einer Hüftoperation zur Präparation des Acetabulums eingesetzt. Dem Acetabulum wird eine halbkugelförmige Ausnehmung im Beckenknochen angepasst, damit eine halbkugelförmige Gelenkschale eines Hüftgelenkes zuverlässig im Becken verankert werden kann. Je genauer das Acetabulum halbkugelförmig präpariert ist, desto formschlüssiger ist der Sitz der Gelenkschale, und desto zuverlässiger ist das Hüftgelenk am Becken verankert.

Zur Präparation des Acetabulums werden die bekannten Instrumente mit dem distalen Ende in den vom Acetabulum begrenzten Raum eingeführt und das Instrument in Drehung versetzt. Idealerweise wird das Instrument so eingeführt, dass die Drehachse, welche zugleich eine Achse der Bearbeitungssphäre ist, mit einer vom Acetabulum definierten Achse zusammenfällt, was als achsparallele Bearbeitung des Acetabulums bezeichnet wird. In der Praxis kann es allerdings geschehen, dass die Drehachse und die Achse des Acetabulums einen, wenn auch kleinen, Winkel miteinander einschließen. Dies liegt beispielsweise daran, dass das Operationsfeld vom Operateur aufgrund eines nur kleinen Einschnitts in den Körper nicht immer perfekt zu erkennen ist. Der Winkel zwischen den Achsen erfordert es, dass bei der Präparation des Acetabulums eine taumelnde Bewegung des Instrumentes vom Operateur ausgeführt werden muss, um den Randbereich des Acetabulums zu präparieren. Dies resultiert jedoch häufig in einer unerwünschten unrunden, d.h. nicht halbkugelförmigen, Präparation des Acetabulums.

Die US 2003/0078587 A1 beschreibt ein chirurgisches Instrument mit den Merkmalen des Oberbegriffs des unabhängigen Anspruchs 1. Das Instrument ist als Acetabulumfräser ausgestaltet.

Ein weiteres, als Acetabulumfräser ausgestaltetes Instrument aus einem Vollmaterial ist in der DE 71 13 620 U1 beschrieben.

Die Erfindung sowie vorteilhafte Ausführungsformen der Erfindung werden nachfolgend aufgrund des vorstehend beschriebenen Einsatzzweckes am Beispiel des Acetabulums erläutert. Dies ist allerdings als nicht limitierend für den Umfang der Erfindung zu verstehen. Nachfolgende Erläuterungen treffen daher ebenfalls für eine Ausnehmung allgemeiner Art in einem Knochen zu. Aufgabe der vorliegenden Erfindung ist es, ein chirurgisches Instrument der eingangs genannten Art so weiterzubilden, dass damit eine bessere Präparation der Ausnehmung im Knochen erzielt werden kann.

Diese Aufgabe wird bei einem gattungsgemäßen chirurgischen Instrument erfindungsgemäß dadurch gelöst, dass das Instrument mehrere proximale Schneidelemente umfasst, deren Schneidkanten in Umfangsrichtung der Drehachse paarweise voneinander beabstandet sind, wobei die Schneidkanten in Umfangsrichtung der Drehachse ungleichmäßig voneinander beabstandet sind.

"Proximales Schneidelement" bezeichnet vorliegend ein Schneidelement, dessen Schneidkante einen proximalseitig bezüglich der Äquatorialebene angeordneten Abschnitt umfasst.

Wird das Acetabulum taumelnd bearbeitet, ist die Äquatorialebene der Bearbeitungssphäre relativ zu einer durch eine Eintrittsöffnung des Acetabulums definierten Eintrittsebene um denselben Winkel verkippt wie die Drehachse relativ zur Achse des Acetabulums. Das mindestens eine proximale Schneidelement kann daher bei taumelnder Bearbeitung in die vom Acetabulum begrenzte Ausnehmung eintauchen. Ein nahe der Eintrittsebene angeordneter Rand des Acetabulums kann dann mit dem mindestens einen proximalen Schneidelement bearbeitet werden. Mittels desselben kann das Instrument während der Taumelbewegung außerdem am Knochen abgestützt werden. In der Praxis lässt sich dadurch eine unrunde Präparation des Acetabulums häufig vermeiden.

Das Instrument umfasst mehrere proximale Schneidelemente. Dadurch kann mit jedem der Schneidelemente einer der vorstehend erläuterten Vorteile erzielt werden. Zusätzlich kann Knochenmaterial verbessert abgetragen werden.

Die Schneidkanten der proximalen Schneidelemente sind in Umfangsrichtung der Drehachse paarweise voneinander beabstandet. Dies erlaubt eine zügigere Präparation des Acetabulums, weil bei der Rotation des Werkzeuges unterschiedliche, in Umfangsrichtung der Achse des Acetabulums angeordnete Knochenbereiche bearbeitet werden können.

Die Schneidkanten der proximalen Schneidelemente sind in Umfangsrichtung der Drehachse ungleichmäßig voneinander beabstandet, so dass die Schneidkanten in Umfangsrichtung der Drehachse nicht äquidistant verteilt sind. In der Praxis kann dadurch ein Verkanten des Instrumentes am Knochen während der Bearbeitung weitgehend vermieden werden.

Das Instrument umfasst einen Grundkörper, an dem die Schneidelemente festgelegt sind. Dies ermöglicht eine einfache konstruktive Ausgestaltung des Instrumentes.

Der Grundkörper ist als Hohlkörper ausgebildet, denn dies spart Material und dadurch Herstellungskosten.

Günstig ist es, wenn sich die Schneidkante mindestens eines proximalen Schneidelementes auf der proximalen Hemisphäre erstreckt, d.h. ebenfalls auf der Bearbeitungssphäre liegt. Dies hat zur Folge, dass beim Eintauchen des mindestens einen proximalen Schneidelementes in den vom Acetabulum begrenzten Raum dessen Rand nahe der Eintrittsebene gemäß der gewünschten Halbkugelform abgetragen wird. Das mindestens eine proximale Schneidelement kann nicht nur Knochenmaterial abtragen, sondern es bildet auch ein Führungselement für das Instrument während der Taumelbewegung. Bei einem bestimmungsgemäßen Gebrauch des Instrumentes führt dies zu einer runden Präparation des Acetabulums.

Es kann vorgesehen sein, dass das Instrument mindestens ein sich auf der proximalen Hemisphäre erstreckendes Führungselement aufweist, das kein proximales Schneidelement ist. Dies ist auch bei einem Instrument der eingangs genannten Art möglich, ein derartiges Instrument kann eine eigene Erfindung darstellen. Mittels des mindestens einen Führungselementes kann sich das Instrument bei taumelnder Bearbeitung an einem Rand des Acetabulums abstützen. Die Erstreckung des mindestens einen Führungsgliedes auf der proximalen Hemisphäre stellt dabei sicher, dass mit den Schneidelementen eine runde Präparation des Acetabulums erzielt werden kann.

Vorzugsweise schließt eine gedachte Verbindungslinie des proximalen Endes der Schneidkante mindestens eines proximalen Schneidelementes mit dem Zentrum der Bearbeitungssphäre einen Winkel von kleiner als ungefähr 15° mit der Äquatorialebene der Bearbeitungssphäre ein, vorzugsweise einen Winkel von ungefähr 5° bis ungefähr 10°. Anders ausgedrückt, erstreckt sich die Schneidkante günstigerweise ungefähr bis zum 15. proximalen Breitengrad der Bearbeitungssphäre, vorzugsweise ungefähr bis zum 5. bis ungefähr zum 10. proximalen Breitengrad. In der Praxis ist eine derartige Erstreckung des mindestens einen proximalen Schneidelementes für eine hinreichend gute Präparation des Acetabulums ausreichend, unter Berücksichtigung desjenigen Winkels, um den die Drehachse relativ zur Achse des Acetabulums üblicherweise verkippt ist.

Vorteilhafterweise erstreckt sich die Schneidkante mindestens eines proximalen Schneidelementes über die Äquatorialebene der Bearbeitungssphäre hinaus in distaler Richtung. Dies erlaubt es, mit dem Instrument das Acetabulum auch bei achsparalleler Präparation halbkugelförmig zu bearbeiten. Derjenige Abschnitt der Schneidkante, der sich ausgehend von der Äquatorialebene in distaler Richtung erstreckt, kann bei der achsparallelen Bearbeitung nämlich zum Abtragen des nahe der Eintrittsebene angeordneten Randes des Acetabulums eingesetzt werden. Somit eignet sich diese Ausführungsform des Instrumentes zu einer runden Präparation des Acetabulums sowohl bei achsparalleler als auch bei taumelnder Bearbeitung.

Bevorzugt schließt eine gedachte Verbindungslinie des distalen Endes der Schneidkante mindestens eines proximalen Schneidelementes mit dem Zentrum der Bearbeitungssphäre einen Winkel von kleiner als ungefähr 20° mit der Äquatorialebene der Bearbeitungssphäre ein, vorzugsweise einen Winkel von ungefähr 8° bis ungefähr 16°. Anders ausgedrückt, erstreckt sich die Schneidkante in distaler Richtung günstigerweise bis ungefähr bis zum 20. distalen Breitengrad, vorzugsweise ungefähr bis zum 8. bis ungefähr zum 16. distalen Breitengrad. Damit ist die Schneidkante distal so weit begrenzt, dass sie sich insbesondere nicht bis zum distalen Pol der Bearbeitungssphäre erstreckt. Es hat sich gezeigt, dass durch eine Begrenzung der Schneidkante eines proximalen Schneidelementes unter zusätzlicher Nutzung distaler Schneidelemente, deren Schneidkanten auf der distalen Hemisphäre der Bearbeitungssphäre räumlich begrenzt sind, die Abtragung von Knochenmaterial während der Präparation erleichtert wird.

Bei einer konkreten Ausgestaltung des Instrumentes ist es in der Praxis günstig, wenn mindestens ein proximales Schneidelement symmetrisch bezüglich der Äquatorialebene der Bearbeitungssphäre am Instrument angeordnet ist. Dadurch wird auch sichergestellt, dass der nahe der Eintrittsebene gelegene Rand des Acetabulums zuverlässig bearbeitet wird, und zwar sowohl bei achsparalleler als auch bei taumelnder Bearbeitung.

Die Herstellung des Instrumentes wird vereinfacht, wenn die proximalen Schneidelemente identisch ausgebildet sind.

Günstig ist es, wenn sich die Schneidkante mindestens eines distalen Schneidelementes in proximaler Richtung bis zur Äquatorialebene der Bearbeitungssphäre oder im Wesentlichen bis zur Äquatorialebene erstreckt. Dies gibt die Möglichkeit, auch bei achsparalleler Bearbeitung Knochen von einem nahe der Eintrittsebene gelegenen Rand des Acetabulums abzutragen. Bei dieser Ausführungsform wird daher eine vorteilhafte Präparation des Acetabulums sowohl bei taumelnder als auch bei achsparalleler Bearbeitung erzielt.

Vorzugsweise erstreckt sich die Schneidkante des Schneidelementes in proximaler Richtung über die Äquatorialebene hinaus, wobei sie sich proximalseitig der Äquatorialebene günstigerweise auf der proximalen Hemisphäre erstreckt. Wie im Zusammenhang mit dem erfindungsgemäßen Instrument erläutert, kann in diesem Fall das distale Schneidelement bei taumelnder Bearbeitung zur Abstützung des Instruments am nahe der Eintrittsebene gelegenen Rand des Acetabulums, zu dessen Abtragung und damit zu einer verbesserten Präparation des Acetabulums eingesetzt werden.

Die vorstehende Erläuterung vorteilhafter Ausführungsformen der Erfindung zeigt, dass vorgesehen sein kann, dass mindestens ein Schneidelement eine Schneidkante sowohl mit einem distalen als auch mit einem proximalen Abschnitt umfasst.

Von Vorteil ist es daher, wenn mindestens ein distales Schneidelement ein proximales Schneidelement bildet und umgekehrt. Ein derartiges Schneidelement weist eine sich beidseitig über die Äquatorialebene hinaus erstreckende Schneidkante auf und kann daher zuverlässig bei achsparalleler und taumelnder Bearbeitung eingesetzt werden.

Es kann vorgesehen sein, dass das Instrument mehrere distale Schneidelemente umfasst.

Die Herstellung des Instruments wird vereinfacht, wenn die distalen Schneidelemente identisch ausgebildet sind.

Eine noch einfachere konstruktive Ausgestaltung weist das Instrument auf, wenn alle Schneidelemente des Instrumentes identisch ausgebildet sind.

Es kann auch vorgesehen sein, dass die Schneidelemente unterschiedlich ausgebildet sind, wobei Schneidelemente im Bereich der Äquatorialebene längere Schneidkanten aufweisen können als Schneidelemente auf der distalen Bearbeitungssphäre, insbesondere nahe deren distalem Pol.

Vorzugsweise verlaufen die Schneidkanten der Schneidelemente des Instrumentes jeweils längs eines Meridians der Bearbeitungssphäre. Dies ermöglicht eine verbesserte Abtragung von Knochenmaterial.

Als günstig hat es sich erwiesen, wenn die Schneidkanten der Schneidelemente des Instrumentes ungefähr 10 bis ungefähr 30 Breitengrade der Bearbeitungssphäre überdecken, bevorzugt ungefähr 20 Breitengrade. Dies hat sich in der Praxis als zuverlässig für die Abtragung von Knochenmaterial herausgestellt.

Es kann auch vorgesehen sein, dass der Winkel zwischen gedachten Verbindungslinien der proximalen und der distalen Enden der Schneidkanten der Schneidelemente des Instruments mit dem Zentrum der Bearbeitungssphäre ungefähr 10° bis ungefähr 30° beträgt, bevorzugt ungefähr 20°. Wie bei der zuletzt genannten Ausführungsform hat sich dies in der Praxis als zuverlässig für die Abtragung von Knochenmaterial erwiesen.

Eine einfache konstruktive Ausgestaltung des Instrumentes wird auch bei einer Ausführungsform erzielt, bei der die Schneidelemente einstückig mit dem Grundkörper ausgebildet sind. Dies vereinfacht die Herstellung des Instrumentes.

Vorzugsweise sind die Schneidelemente als Vorsprünge des Grundkörpers in radialer Richtung, bezogen auf das Zentrum der Bearbeitungssphäre, ausgebildet. Dies ermöglicht eine einfache konstruktive Ausgestaltung des Instrumentes, insbesondere wenn die Schneidelemente einstückig mit dem Grundkörper ausgebildet sind. Die Schneidelemente können beispielsweise um circa 0,5 mm bis circa 4 mm vorspringen und bevorzugt um circa 1 mm bis circa 3 mm.

Bevorzugt umfasst der Grundkörper im Bereich eines Schneidelementes eine Materialausnehmung zur Aufnahme abgetragenen Knochenmaterials. Dies ermöglicht eine zuverlässigere Funktion des Instrumentes. Knochenmaterial kann in die Aufnahme eintreten, so dass es sich bei der Bearbeitung des Acetabulums nicht störend auswirkt.

Günstig ist es, wenn die Materialausnehmung als Durchbrechung des Grundkörpers ausgebildet ist, insbesondere wenn dieser als Hohlkörper ausgebildet ist. Dadurch kann das Knochenmaterial in den vom Hohlkörper zumindest teilweise eingefassten Hohlraum eintreten und während des Einsatzes des Instrumentes besonders gut abgeführt werden.

Bei einer Umsetzung des Instrumentes in der Praxis hat es sich als günstig erwiesen, wenn die Materialausnehmung ungefähr 10 bis ungefähr 30 Breitengrade der Bearbeitungssphäre, vorzugsweise ungefähr 20 Breitengrade, überdeckt und/oder wenn die Materialausnehmung ungefähr 10 bis ungefähr 30 Längengrade der Bearbeitungssphäre, vorzugsweise ungefähr 20 Längengrade, überdeckt. Dadurch kann eine zuverlässige Abfuhr von Knochenmaterial erzielt werden.

Vorteilhafterweise weist der Grundkörper eine sphärische Außenkontur auf. Dies ermöglicht eine einfache konstruktive Ausgestaltung des Instrumentes. Auch als Vorsprünge des Grundkörpers ausgebildete Schneidelemente lassen sich dadurch auf einfache Weise herstellen. Ein als Hohlkörper ausgebildeter Grundkörper kann beispielsweise die Gestalt einer Kugelschale mit sphärischer Außenkontur haben.

Vorzugsweise umfasst der Grundkörper einen über die Äquatorialebene der Bearbeitungssphäre in proximaler Richtung hinaus ragenden Randbereich. Der Randbereich kann damit einen Abschnitt des Grundkörpers proximalseitig der Äquatorialebene bilden, an dem mindestens ein proximales Schneidelement festgelegt ist, beispielsweise ist dieses als Vorsprung des Randbereiches ausgebildet.

Der Randbereich weist günstigerweise ebenfalls eine sphärische Außenkontur auf, welche eine distalseitig der Äquatorialebene liegende sphärische Außenkontur des Grundkörpers insbesondere stetig fortsetzt.

Vorteilhafterweise schließt eine gedachte Verbindungslinie des proximalen Endes des Randbereiches mit dem Zentrum der Bearbeitungssphäre einen Winkel mit der Äquatorialebene von ungefähr 5° bis ungefähr 20° ein, bevorzugt ungefähr 10° bis ungefähr 18°. Bei einer Umsetzung des Instrumentes erweist sich eine derartige Größe des Randbereichs als ausreichend zum Tragen bzw. Festlegen des mindestens einen proximalen Schneidelementes.

Günstig ist es, wenn der Randbereich in proximaler Richtung über das proximale Ende der Schneidkante mindestens eines proximalen Schneidelementes hinaus ragt. Dies verringert die Gefahr, dass sich der Operateur an der Schneidkante verletzt. Ferner ist insbesondere beim Einbringen des Instrumentes in den Körper die Gefahr verringerbar, dass die Schneidkante zu einer Verletzung des Patienten führt.

Es kann vorgesehen sein, dass der Randbereich einen parallel zur Äquatorialebene der Bearbeitungssphäre verlaufenden freien Rand aufweist.

Bei einer vorteilhaften Ausführungsform des erfindungsgemäßen Instrumentes hat es sich als günstig erwiesen, wenn der Randbereich in Umfangsrichtung der Drehachse zusammenhängend ausgebildet ist, d.h. der Randbereich ist in Umfangsrichtung der Drehachse in sich geschlossen. Dies ermöglicht es beispielsweise, dem Randbereich und damit dem Grundkörper eine erhöhte Stabilität zu verleihen.

Zur Erzielung einer einfachen Konstruktion ist der Randbereich in Umfangsrichtung der Drehachse vorteilhafterweise einstückig ausgebildet.

Bei einer andersartigen Ausführungsform kann vorgesehen sein, dass der Randbereich in Umfangsrichtung der Drehachse zumindest eine Unterbrechung aufweist.

Bei einer weiteren Ausführungsform des erfindungsgemäßen Instrumentes ist es günstig, wenn der Randbereich in Umfangsrichtung der Drehachse in zwei oder mehr voneinander beabstandete Randsegmente unterteilt ist, so dass zwischen je zwei Randsegmenten Ausnehmungen angeordnet sind. Dadurch kann beispielsweise Material eingespart werden. Der vorstehend genannte freie Rand kann dann im Bereich der Ausnehmungen unterbrochen sein.

Vorzugsweise ist innerhalb des von einem Randsegment überdeckten Winkelbereiches in Umfangsrichtung der Drehachse mindestens ein proximales

Schneidelement angeordnet, d.h. das Randsegment ist zumindest teilweise Träger mindestens eines proximalen Schneidelementes. Dies gibt die Möglichkeit, den Randbereich nicht größer als erforderlich auszubilden, so dass, wie vorstehend erwähnt, Material eingespart werden kann.

Vorzugsweise weisen die Ausnehmungen jeweils einen in der Äquatorialebene angeordneten Boden auf. Anhand der Böden der Ausnehmungen kann der Operateur erkennen, wo die Äquatorialebene liegt. Bei der Präparation des Acetabulums ist dies für den Operateur sehr hilfreich, weil die Böden gewissermaßen eine Markierung der Äquatorialebene bilden. Dadurch kann der Operateur anhand der Böden der Ausnehmung zum Einen erkennen, ob er taumelnd oder achsparallel präpariert, und zum Anderen kann die Eindringtiefe des Instrumentes in den Knochen überwacht werden. Dies erlaubt es dem Operateur, das Instrument nur so lange mit einer distal wirksamen Kraft zu beaufschlagen, bis die Böden der Ausnehmungen in der Eintrittsebene des Acetabulums liegen. Daran kann der Operateur erkennen, dass das Acetabulum halbkugelförmig präpariert ist.

Bei der vorstehend erwähnten Ausführungsform des mit einer Unterbrechung in Umfangsrichtung der Drehachse versehenen Randbereiches kann in entsprechender Weise vorgesehen sein, dass die Unterbrechung eine Ausnehmung vom Randbereich definiert, die einen in der Äquatorialebene der Bearbeitungssphäre liegenden Boden aufweist.

Bei einer Umsetzung des Instrumentes in der Praxis ist es günstig, wenn die zwei oder mehr Randsegmente in Umfangsrichtung der Drehachse jeweils einen Winkelbereich von ungefähr 20° bis ungefähr 40° überdecken, bevorzugt einen Winkelbereich von ungefähr 30°. Dies gibt die Möglichkeit, das mindestens eine proximale Schneidelement sowie gegebenenfalls eine Materialausnehmung für Knochenmaterial mit den vorstehend genannten Abmessungen bzw. Winkelerstreckungen an einem Randsegment auszubilden.

Bei einer konstruktiv einfachen Ausgestaltung, die die Herstellung des Instrumentes erleichtert, sind die zwei oder mehr Randsegmente zinnenförmig ausgestaltet, insbesondere in Form von Rechteckzinnen. Die Lücken zwischen den Zinnen definieren dann die vorstehend genannten Ausnehmungen.

Eine einfachere Herstellung des Instrumentes wird ermöglicht, wenn die zwei oder mehr Randsegmente identisch ausgebildet sind.

Wie bereits erwähnt, ist es von Vorteil, wenn der Operateur den Äquator der Bearbeitungssphäre erkennen kann. Deswegen ist es günstig, wenn der Grundkörper eine Markierung umfasst, anhand derer der Äquator der Bearbeitungssphäre erkennbar ist. Diese Markierung kann beispielsweise in Form eines leicht erkennbaren farblichen Streifens ausgebildet sein. Es ist auch möglich, dass die Markierung als Nut im Grundkörper oder als vorspringende Rippe des Grundkörpers ausgestaltet ist.

Bei einer andersartigen Ausführungsform des Instrumentes kann vorgesehen sein, dass das Instrument ein den Äquator kennzeichnendes Markierungselement aufweist, das nicht vom Grundkörper umfasst ist.

Von Vorteil ist es, wenn das Instrument eine Verbindungseinrichtung zum Verbinden mit einer Antriebseinrichtung aufweist. Dadurch kann das Instrument drehend angetrieben werden.

Eine besonders einfache Konstruktion des Instrumentes kann erzielt werden, wenn es einstückig ausgebildet ist.

Wie sich bereits aus der vorstehenden Beschreibung ergibt, kann ein erfindungsgemäßes Instrument speziell zur Präparation des Acetabulums eingesetzt werden. Daher ist es günstig, wenn das Instrument ein Acetabulumfräskopf oder ein Acetabulumfräser ist. Der Fräskopf oder Fräser weist insbesondere eine Verbindungseinrichtung auf, mit der er lösbar mit einem von einer Antriebseinrichtung drehend angetriebenen Schaft verbunden werden kann.

Die Erfindung betrifft daher auch ein chirurgisches Instrumentarium, umfassend mindestens eines der vorstehend genannten Instrumente sowie eine Antriebseinrichtung, wobei das mindestens eine Instrument lösbar mit der Antriebseinrichtung verbindbar und von dieser drehend antreibbar ist.

Mit einem erfindungsgemäßen chirurgischen Instrumentarium kann ebenfalls eine bessere Präparation der Ausnehmung im Knochen erzielt werden.

Die vorstehende Beschreibung umfasst somit insbesondere die nachfolgend in Form durchnummerierter Sätze definierten Ausführungsformen eines chirurgischen Instrumentes und eines chirurgischen Instrumentariums:
1. Chirurgisches Instrument zur Erzeugung einer halbkugelförmigen Ausnehmung (12) in einem Knochen (14), das um eine Drehachse (28) drehend antreibbar ist und mindestens ein distales Schneidelement (36) mit einer Schneidkante (38) zur Abtragung von Knochenmaterial umfasst, wobei die Schneidkante (38) in Rotation des Instrumentes (10; 100) um die Drehachse (28) eine Bearbeitungssphäre (B) mit einer in die Ausnehmung (12) einführbaren distalen Hemisphäre, einem Äquator und einer proximalen Hemisphäre definiert, dadurch gekennzeichnet, dass das Instrument (10; 100) mindestens ein proximales Schneidelement (46, 48, 50; 146, 148, 150) mit einer Schneidkante (38) umfasst, die sich ausgehend von der Äquatorialebene (A) der Bearbeitungssphäre (B) oder im Wesentlichen von der Äquatorialebene (A) in proximaler Richtung erstreckt.
2. Instrument nach Satz 1, dadurch gekennzeichnet, dass sich die Schneidkante (38) des mindestens einen proximalen Schneidelementes (46, 48, 50; 146, 148, 150) auf der proximalen Hemisphäre erstreckt.
3. Instrument nach Satz 1 oder 2, dadurch gekennzeichnet, dass eine gedachte Verbindungslinie des proximalen Endes der Schneidkante (38) des mindestens einen proximalen Schneidelementes (46, 48, 50; 146, 148, 150) mit dem Zentrum (Z) der Bearbeitungssphäre (B) einen Winkel von kleiner als ungefähr 15° mit der Äquatorialebene (A) der Bearbeitungssphäre (B) einschließt, vorzugsweise einen Winkel von ungefähr 5° bis ungefähr 10°.
4. Instrument nach einem der voranstehenden Sätze, dadurch gekennzeichnet, dass sich die Schneidkante (38) des mindestens einen proximalen Schneidelementes (46, 48, 50; 146, 148, 150) über die Äquatorialebene (A) der Bearbeitungssphäre (B) hinaus in distaler Richtung erstreckt.
5. Instrument nach Satz 4, dadurch gekennzeichnet, dass eine gedachte Verbindungslinie des distalen Endes der Schneidkante (38) des mindestens einen proximalen Schneidelementes (46, 48, 50; 146, 148, 150) mit dem Zentrum (Z) der Bearbeitungssphäre (B) einen Winkel von kleiner als ungefähr 20° mit der Äquatorialebene (A) der Bearbeitungssphäre (B) einschließt, vorzugsweise einen Winkel von ungefähr 8° bis ungefähr 16°.
6. Instrument nach Satz 4 oder 5, dadurch gekennzeichnet, dass das mindestens eine proximale Schneidelement (146, 148, 150) symmetrisch bezüglich der Äquatorialebene (A) der Bearbeitungssphäre (B) am Instrument (10; 100) angeordnet ist.
7. Instrument nach einem der voranstehenden Sätze, dadurch gekennzeichnet, dass das Instrument (10; 100) mehrere proximale Schneidelemente (46, 48, 50; 146, 148, 150) umfasst.
8. Instrument nach Satz 7, dadurch gekennzeichnet, dass die Schneidkanten (38) der proximalen Schneidelemente (46, 48, 50; 146, 148, 150) in Umfangsrichtung der Drehachse (28) paarweise voneinander beabstandet sind.
9. Instrument nach Satz 8, dadurch gekennzeichnet, dass die Schneidkanten (38) der proximalen Schneidelemente (46, 48, 50; 146, 148, 150) in Umfangsrichtung der Drehachse (28) ungleichmäßig voneinander beabstandet sind.
10. Instrument nach einem der Sätze 7 bis 9, dadurch gekennzeichnet, dass die proximalen Schneidelemente (46, 48, 50; 146, 148, 150) identisch ausgebildet sind.
11. Instrument nach einem der voranstehenden Sätze oder nach dem Oberbegriff von Satz 1, dadurch gekennzeichnet, dass sich die Schneidkante (38) mindestens eines distalen Schneidelementes (36) in proximaler Richtung bis zur Äquatorialebene (A) der Bearbeitungssphäre (B) oder im Wesentlichen bis zur Äquatorialebene (A) erstreckt.
12. Instrument nach Satz 11, dadurch gekennzeichnet, dass sich die Schneidkante (38) des Schneidelementes (36) in proximaler Richtung über die Äquatorialebene (A) hinaus erstreckt.
13. Instrument nach einem der voranstehenden Sätze, dadurch gekennzeichnet, dass mindestens ein distales Schneidelement (36) ein proximales Schneidelement (46, 48, 50; 146, 148, 150) bildet und umgekehrt.
14. Instrument nach einem der voranstehenden Sätze, dadurch gekennzeichnet, dass das Instrument (10; 100) mehrere distale Schneidelemente (36) umfasst.
15. Instrument nach Satz 14, dadurch gekennzeichnet, dass die distalen Schneidelemente (36) identisch ausgebildet sind.
16. Instrument nach einem der voranstehenden Sätze, dadurch gekennzeichnet, dass alle Schneidelemente (36, 46, 48, 50; 146, 148, 150) des Instrumentes (10; 100) identisch ausgebildet sind.
17. Instrument nach einem der voranstehenden Sätze, dadurch gekennzeichnet, dass die Schneidkanten (38) der Schneidelemente (36, 46, 48, 50; 146, 148, 150) des Instrumentes (10; 100) jeweils längs eines Meridians der Bearbeitungssphäre (B) verlaufen.
18. Instrument nach einem der voranstehenden Sätze, dadurch gekennzeichnet, dass die Schneidkanten (38) der Schneidelemente (36, 46, 48, 50; 146, 148, 150) des Instrumentes (10; 100) ungefähr 10 bis ungefähr 30 Breitengrade der Bearbeitungssphäre (B) überdecken, bevorzugt ungefähr 20 Breitengrade.
19. Instrument nach einem der voranstehenden Sätze, dadurch gekennzeichnet, dass der Winkel zwischen gedachten Verbindungslinien der proximalen und der distalen Enden der Schneidkanten (38) der Schneidelemente (36, 46, 48, 50; 146, 148, 150) des Instrumentes (10; 100) mit dem Zentrum (Z) der Bearbeitungssphäre (B) ungefähr 10° bis ungefähr 30° beträgt, bevorzugt ungefähr 20°.
20. Instrument nach einem der voranstehenden Sätze, dadurch gekennzeichnet, dass das Instrument (10; 100) einen Grundkörper (18) umfasst, an dem die Schneidelemente (36, 46, 48, 50; 146, 148, 150) festgelegt sind.
21. Instrument nach Satz 20, dadurch gekennzeichnet, dass die Schneidelemente (36, 46, 48, 50; 146, 148, 150) einstückig mit dem Grundkörper (18) ausgebildet sind.
22. Instrument nach Satz 20 oder 21, dadurch gekennzeichnet, dass die Schneidelemente (36, 46, 48, 50; 146, 148, 150) als Vorsprünge (34) des Grundkörpers (18) in radialer Richtung, bezogen auf das Zentrum (Z) der Bearbeitungssphäre (B), ausgebildet sind.
23. Instrument nach einem der Sätze 20 bis 22, dadurch gekennzeichnet, dass der Grundkörper (18) als Hohlkörper ausgebildet ist.
24. Instrument nach einem der Sätze 20 bis 23, dadurch gekennzeichnet, dass der Grundkörper (18) im Bereich eines Schneidelementes (36, 46, 48, 50; 146, 148, 150) eine Materialausnehmung (40) zur Aufnahme abgetragenen Knochenmaterials umfasst.
25. Instrument nach Satz 24, dadurch gekennzeichnet, dass die Materialausnehmung (40) als Durchbrechung (42) des Grundkörpers (18) ausgebildet ist.
26. Instrument nach Satz 24 oder 25, dadurch gekennzeichnet, dass die Materialausnehmung (40) ungefähr 10 bis ungefähr 30 Breitengrade der Bearbeitungssphäre (B), vorzugsweise ungefähr 20 Breitengrade, überdeckt und/oder dass die Materialausnehmung (40) ungefähr 10 bis ungefähr 30 Längengrade der Bearbeitungssphäre (B), vorzugsweise ungefähr 20 Längengrade, überdeckt.
27. Instrument nach einem der Sätze 20 bis 26, dadurch gekennzeichnet, dass der Grundkörper (18) eine sphärische Außenkontur aufweist.
28. Instrument nach einem der Sätze 20 bis 27, dadurch gekennzeichnet, dass der Grundkörper (18) einen über die Äquatorialebene (A) der Bearbeitungssphäre (B) in proximaler Richtung hinaus ragenden Randbereich (30; 130) umfasst.
29. Instrument nach Satz 28, dadurch gekennzeichnet, dass eine gedachte Verbindungslinie des proximalen Endes des Randbereiches (30; 130) mit dem Zentrum (Z) der Bearbeitungssphäre (B) einen Winkel mit der Äquatorialebene (A) von ungefähr 5° bis ungefähr 20° einschließt, bevorzugt ungefähr 10° bis ungefähr 18°.
30. Instrument nach Satz 28 oder 29, dadurch gekennzeichnet, dass der Randbereich (30; 130) in proximaler Richtung über das proximale Ende der Schneidkante (38) des mindestens einen proximalen Schneidelementes (46, 48, 50; 146, 148, 150) hinaus ragt.
31. Instrument nach einem der Sätze 28 bis 30, dadurch gekennzeichnet, dass der Randbereich (30; 130) einen parallel zur Äquatorialebene (A) der Bearbeitungssphäre (B) verlaufenden freien Rand (32; 134) aufweist.
32. Instrument nach einem der Sätze 28 bis 31, dadurch gekennzeichnet, dass der Randbereich (30) in Umfangsrichtung der Drehachse (28) zusammenhängend ausgebildet ist.
33. Instrument nach Satz 32, dadurch gekennzeichnet, dass der Randbereich (30) in Umfangsrichtung der Drehachse (28) einstückig ausgebildet ist.
34. Instrument nach einem der Sätze 28 bis 31, dadurch gekennzeichnet, dass der Randbereich (130) in Umfangsrichtung der Drehachse (28) in zwei oder mehr voneinander beabstandete Randsegmente (132) unterteilt ist, so dass zwischen je zwei Randsegmenten (132) Ausnehmungen (154) angeordnet sind.
35. Instrument nach Satz 34, dadurch gekennzeichnet, dass innerhalb des von einem Randsegment (132) überdeckten Winkelbereiches in Umfangsrichtung der Drehachse (28) mindestens ein proximales Schneidelement (46, 48, 50; 146, 148, 150) angeordnet ist.
36. Instrument nach Satz 34 oder 35, dadurch gekennzeichnet, dass die Ausnehmungen (154) jeweils einen in der Äquatorialebene (A) angeordneten Boden (156) aufweisen.
37. Instrument nach einem der Sätze 34 bis 36, dadurch gekennzeichnet, dass die zwei oder mehr Randsegmente (132) in Umfangsrichtung der Drehachse (28) jeweils einen Winkelbereich von ungefähr 20° bis ungefähr 40° überdecken, bevorzugt einen Winkelbereich von ungefähr 30°.
38. Instrument nach einem der Sätze 34 bis 37, dadurch gekennzeichnet, dass die zwei oder mehr Randsegmente (132) zinnenförmig ausgestaltet sind.
39. Instrument nach einem der Sätze 34 bis 38, dadurch gekennzeichnet, dass die zwei oder mehr Randsegmente (132) identisch ausgebildet sind.
40. Instrument nach einem der Sätze 20 bis 39, dadurch gekennzeichnet, dass der Grundkörper (18) eine Markierung (54; 158) umfasst, anhand derer der Äquator der Bearbeitungssphäre (B) erkennbar ist.
41. Instrument nach einem der voranstehenden Sätze, dadurch gekennzeichnet, dass das Instrument (10; 100) eine Verbindungseinrichtung zum Verbinden mit einer Antriebseinrichtung (60) umfasst.
42. Instrument nach einem der voranstehenden Sätze, dadurch gekennzeichnet, dass das Instrument (10; 100) einstückig ausgebildet ist.
43. Instrument nach einem der voranstehenden Sätze, dadurch gekennzeichnet, dass das Instrument (10; 100) ein Acetabulumfräskopf oder ein Acetabulumfräser ist.
44. Chirurgisches Instrumentarium, umfassend mindestens ein Instrument (10; 100) nach einem der vorstehenden Sätze sowie eine Antriebseinrichtung (60), wobei das mindestens eine Instrument (10; 100) lösbar mit der Antriebseinrichtung (60) verbindbar und von dieser drehend antreibbar ist.

Die nachfolgende Beschreibung bevorzugter Ausführungsformen der Erfindung dient im Zusammenhang mit der Zeichnung der näheren Erläuterung der Erfindung. Es zeigen:
- Figur 1:: eine perspektivische Ansicht einer ersten bevorzugten Ausführungsform eines erfindungsgemäßen chirurgischen Instruments;
- Figur 2:: eine schematische Seitenansicht, teilweise geschnitten, des Instrumentes aus Figur 1 bei achsparalleler Präparation des Acetabulums;
- Figur 3:: eine Darstellung entsprechend Figur 2 bei taumelnder Präparation des Acetabulums;
- Figur 4:: eine Darstellung entsprechend Figur 3, wobei das Acetabulum mit einer zweiten bevorzugten Ausführungsform eines erfindungsgemäßen Instrumentes präpariert wird und
- Figur 5:: ein erfindungsgemäßes chirurgisches Instrumentarium.

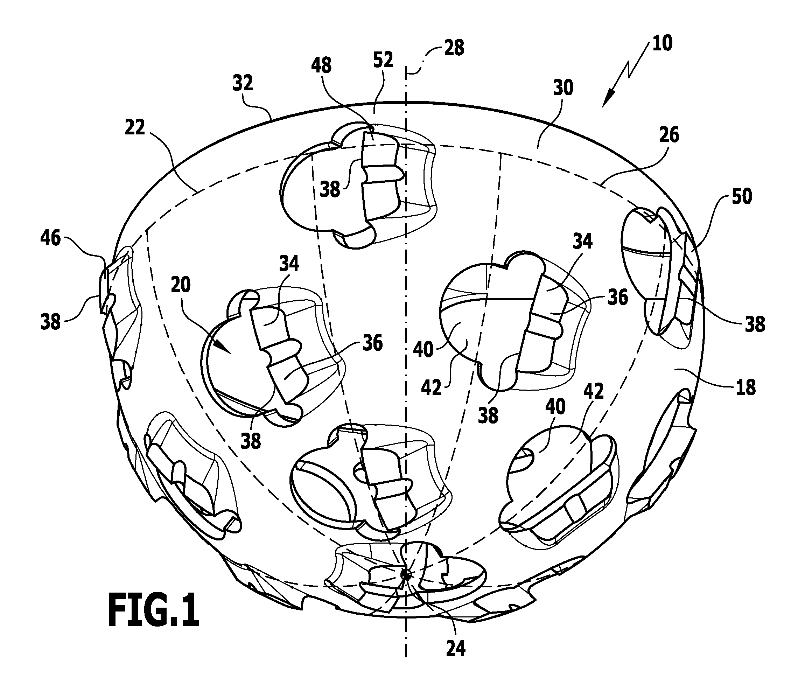

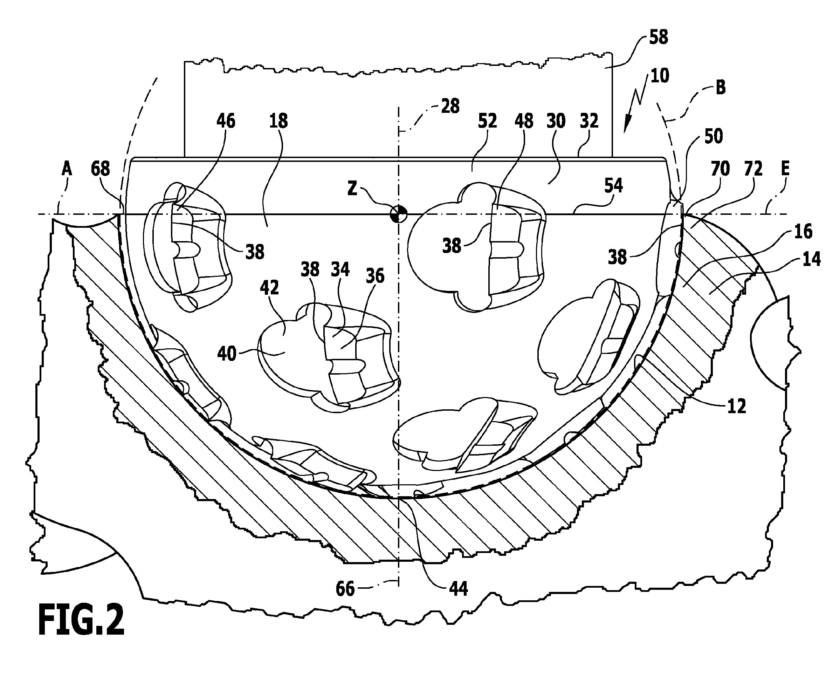

Figur 1 zeigt in perspektivischer Darstellung eine erste bevorzugte Ausführungsform eines erfindungsgemäßen chirurgischen Instrumentes, das vorliegend als Fräskopf 10 ausgestaltet ist. Der Fräskopf 10 dient zur Erzeugung einer kugelförmigen, insbesondere halbkugelförmigen Ausnehmung 12 in einem in den Figuren 2 bis 4 schematisch dargestellten Knochen 14. Bei dem Knochen 14 kann es sich beispielsweise um das menschliche Becken handeln, speziell dessen Acetabulum 16. Das Acetabulum 16 ist mittels des Fräskopfes 10 so zu präparieren, dass die Ausnehmung 12 halbkugelförmig ist, so dass eine kugelschalenförmige Gelenkpfanne eines künstlichen Hüftgelenkes darin formschlüssig und zuverlässig verankert werden kann.

Der Fräskopf 10 ist einstückig ausgebildet und aus Metall gefertigt. Er umfasst einen Grundkörper 18 von kugelschalenförmiger Gestalt, so dass der Grundkörper 18 eine sphärische Außenkontur aufweist und einen Hohlkörper bildet, der ungefähr halbseitig einen Hohlraum 20 einfasst.

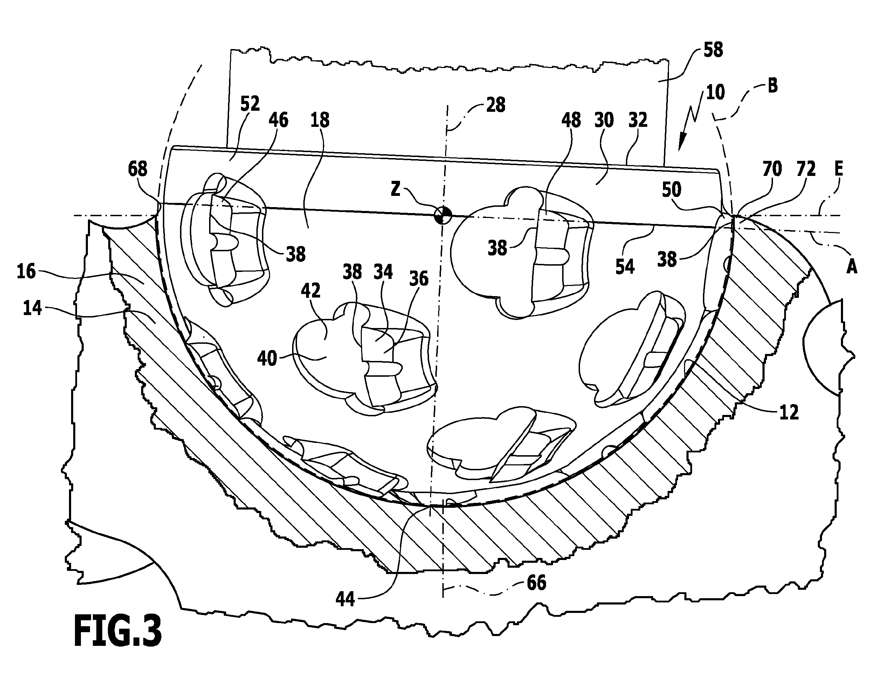

Aufgrund der kugelschalenförmigen Ausgestaltung kann dem Grundkörper 18 ein Koordinatensystem 22 zugewiesen werden, welches in Figur 1 zeichnerisch über den Fräskopf 10 gelegt ist. Das Koordinatensystem 22 zeigt einen Pol 24 des Grundkörpers 18 sowie dessen Äquator 26 und ferner eine Achse 28, welche eine vom Äquator 26 definierte Äquatorialebene A (Figuren 2 und 3) rechtwinklig schneidet.

Nur näherungsweise ist der Grundkörper 18 halbschalenförmig ausgestaltet. Er weist nämlich einen über die Äquatorialebene A in proximaler Richtung überstehenden Randbereich 30 auf mit einem parallel zur Äquatorialebene A verlaufenden freien Rand 32. "Proximal" ist vorliegend in Bezug auf einen den Fräskopf 10 einsetzenden Operateur zu beziehen. Der dem Randbereich 30, bezüglich der Äquatorialebene A, gegenüberliegende Abschnitt des Grundkörpers 18 wird als "distaler" Abschnitt des Grundkörpers 18 bezeichnet. Der distale Abschnitt taucht bei der Präparation des Acetabulums 16 in die von diesem definierte Ausnehmung 12 ein.

Der Randbereich 30 ragt so weit über die Äquatorialebene A hinaus, dass er sich, bezogen auf das vom Grundkörper 18 definierte kugelförmige Koordinatensystem 22, ungefähr bis zum 12. Breitengrad erstreckt. In Umfangsrichtung der Achse 28 ist der Randbereich 30 zusammenhängend und ohne Unterbrechungen ausgebildet.

Der gedachte Schnittpunkt der Achse 28 mit der Äquatorialebene A definiert ein Zentrum Z der vom Grundkörper 18 definierten Kugelschale. In radialer Richtung, bezogen auf dieses Zentrum Z, springen aus dem Grundkörper 18 eine Vielzahl von Vorsprüngen 34 hervor, die allesamt identisch ausgebildet sind. Die Vorsprünge 34 bilden Schneidelemente 36 des Fräskopfes 10, welche jeweils eine scharf zulaufende Schneidkante 38 umfassen. Mittels der Schneidkanten 38, dies wird nachher noch erläutert, kann Knochenmaterial vom Knochen 14 abgetragen werden.

Die Schneidelemente 36 sind derart ausgebildet, dass die Schneidkanten 38 bei Rotation des Fräskopfes 10 um die Achse 28 gemeinsam eine nachfolgend als Bearbeitungssphäre B bezeichnete Sphäre definieren (Figuren 2 bis 4). Der Durchmesser der Bearbeitungssphäre B ist geringfügig größer als der derjenigen Sphäre, die vom Grundkörper 18 definiert wird, weil die Schneidelemente 36, wie erwähnt, in radialer Richtung geringfügig aus dem Grundkörper 18 hervorspringen.

Die vom Grundkörper 18 definierte Sphäre und die Bearbeitungssphäre B sind konzentrisch bezüglich des Zentrums Z gebildet. Aus diesem Grund weist die Bearbeitungssphäre B eine mit der Achse 28 zusammenfallende Achse auf, und die Äquatorialebene A des Grundkörpers 18 fällt mit einer Äquatorialebene der Bearbeitungssphäre B zusammen. Letztere Äquatorialebene wird aus diesem Grund nachfolgend ebenfalls als Äquatorialebene A bezeichnet, und als Achse der Bearbeitungssphäre B wird ebenfalls die Achse 28 bezeichnet.

Die Schneidkanten 38 verlaufen jeweils längs eines Meridians der Bearbeitungssphäre B, wobei sie einen Winkel von ungefähr 20 Breitengraden der Bearbeitungssphäre B überdecken. Diese hat sich bei einer Rotation des Fräskopfes 10 um die Achse 28 als zuverlässig für die Abtragung von Knochenmaterial des Knochens 14 herausgestellt.

Jedem Schneidelement 36 unmittelbar benachbart ist im Grundkörper 18 eine Materialausnehmung 40 in Form einer Durchbrechung 42 des Grundkörpers 18 angeordnet. Die Durchbrechungen 42 erstrecken sich jeweils ungefähr über 20 Breitengrade und ungefähr über 20 Längengrade der Bearbeitungssphäre B. Beim Bearbeiten des Knochens 14 mit dem Fräskopf 10 kann abgetragenes Knochenmaterial durch die Durchbrechungen 42 hindurch in den vom Grundkörper 18 eingefassten Hohlraum 20 gelangen und dadurch zuverlässig vom Bearbeitungsgebiet abgeführt werden.

Jedes Schneidelement 38 ist in sich symmetrisch ausgestaltet, und die Mitten der Schneidkanten 38 liegen auf einer in der Zeichnung nicht dargestellten gedachten Spirallinie, die sich ausgehend von einem Pol 44 der Bearbeitungssphäre B über deren Oberfläche erstreckt. Da die Schneidkanten 38, wie bereits erwähnt, jeweils ungefähr 20 Breitengrade der Bearbeitungssphäre B überdecken, wird dadurch eine flächendeckende Bearbeitung des Knochens 14 ermöglicht.

Die Schneidelemente 36 können eingeteilt werden in zwei sich nicht gegenseitig ausschließende Gruppen, und zwar in "distale" Schneidelemente und "proximale" Schneidelemente. Ein distales Schneidelement 36 ist derart am Grundkörper 18 angeordnet, dass es einen sich distalseitig bezüglich der Äquatorialebene A angeordneten Abschnitt seiner Schneidkante 38 aufweist. Ein proximalseitiges Schneidelement 36 ist am Grundkörper 18 derart angeordnet, dass es einen sich proximalseitig bezüglich der Äquatorialebene A angeordneten Abschnitt seiner Schneidkante 38 aufweist.

Demzufolge umfasst der Fräskopf 10 eine Mehrzahl von rein distalen Schneidelementen 36, deren Schneidkanten 38 gänzlich distal bezüglich der Äquatorialebene A angeordnet sind und auf einer distalen Hemisphäre der Bearbeitungssphäre B liegen. Insbesondere aber umfasst der Fräskopf 10 fünf proximale Schneidelemente 36, von denen in der Zeichnung nur drei proximale Schneidelemente 46, 48 und 50 zu sehen sind (Figuren 1 bis 3). Die Schneidkanten 38 der proximalen Schneidelemente 46 bis 50 ragen sowohl in proximaler als auch in distaler Richtung jeweils über die Äquatorialebene A hinaus, so dass es sich bei den proximalen Schneidelementen 46 bis 50 zusätzlich ebenfalls um distale Schneidelemente handelt.

Die proximalen Schneidelemente 46 bis 50 weisen also jeweils sowohl einen sich proximalseitig als auch einen sich distalseitig der Äquatorialebene A befindenden Abschnitt ihrer Schneidkanten 38 auf. Dabei erstrecken sich die Schneidkanten 38 der proximalen Schneidelemente 46 bis 50 in proximaler Richtung ungefähr bis zum dritten Breitengrad der Bearbeitungssphäre B. In distaler Richtung erstrecken sich die Schneidkanten 38 der proximalen Schneidelemente 46 bis 50 ungefähr bis zum 17. Breitengrad der Bearbeitungssphäre B, also auf der distalen Hemisphäre der Bearbeitungssphäre B.

In Umfangsrichtung der Achse 28 sind die proximalen Schneidelemente 46 bis 50 sowie die nicht dargestellten Schneidelemente voneinander beabstandet, und zwar um ungleichmäßige Winkel bezüglich der Achse 28. Dies verringert die Gefahr, dass sich der Fräskopf 10 beim Bearbeiten des Knochens 14 verkantet und "rattert".

Die Ausgestaltung der proximalen Schneidelemente 46 bis 50 am erfindungsgemäßen Fräskopf 10 wird ermöglicht durch Ausbildung des über die Äquatorialebene A hinaus stehenden Randbereiches 30, als dessen Vorsprünge die Schneidelemente 48 bis 50 zumindest teilweise ausgebildet sind. Der freie Rand 32 überragt dabei in proximaler Richtung die proximalen Enden der Schneidkanten 38 der Schneidelemente 46 bis 50. Zwischen dem freien Rand 32 und den vorgenannten proximalen Enden der Schneidkanten 38 ist daher ein Zwischenabschnitt 52 am Randbereich 30 gebildet, also im Übergangsbereich von den proximalen Enden der Schneidkanten 38 bis zum freien Rand 32. Dies gewährleistet es, dass beim Einbringen des Fräskopfes 10 in den Körper des Patienten die Verletzungsgefahr des Operateurs verringert ist, da er weniger leicht in Kontakt mit den Schneidkanten 38 der Schneidelemente 46 bis 50 geraten kann. Überdies ist die Verletzungsgefahr des Patienten verringert. Dies ist vorliegend auch von Bedeutung, da der Fräskopf 10 in der Regel durch einen relativ geringen Einschnitt in den Körper eingebracht wird. Die vorstehende Ausgestaltung des Fräskopfes 10 verringert daher speziell die Verletzungsgefahr bei Weichteilen des Patienten.

Außerdem weist der Fräskopf 10 noch eine in den Figuren 2 und 3 dargestellte Markierung 54 auf, die den Äquator 26 des Grundkörpers 18 und damit die Äquatorialebene A kennzeichnet. Sie ist für den Operateur erkennbar außenseitig am Grundkörper 18 aufgebracht, z.B. in Form einer farbigen Markierung 54, eines umlaufenden Vorsprungs, einer umlaufenden Nut oder dergleichen.

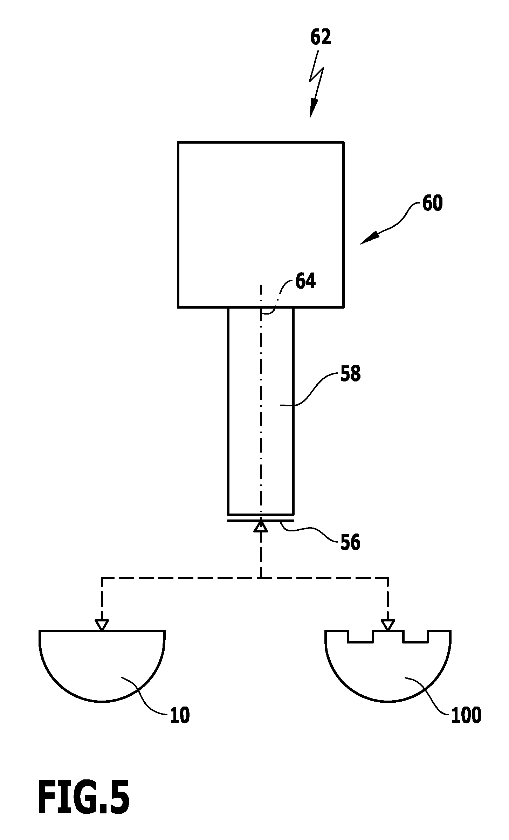

Ferner umfasst der Fräskopf 10 eine in der Zeichnung nicht dargestellte Verbindungseinrichtung, um mit einer korrespondierend dazu ausgebildeten Verbindungseinrichtung 56 eines Schaftes 58 einer in Figur 5 schematisch dargestellten Antriebseinrichtung 60 lösbar verbunden zu werden. Der Fräskopf 10 bildet zusammen mit der Antriebseinrichtung 60 sowie mit einem weiteren Fräskopf 100, auf den nachfolgend noch eingegangen wird, eine bevorzugte Ausführungsform eines erfindungsgemäßen chirurgischen Instrumentariums 62. Der Fräskopf 10 kann mit dem Schaft 58 derart verbunden werden, dass eine Achse 64 des Schaftes 58 mit der Achse 28 zusammenfällt, so dass wahlweise einer der Fräsköpfe 10, 100 um diese gemeinsame Drehachse drehend angetrieben werden kann. Wie erwähnt, ist die Achse 28 auch eine Achse der Bearbeitungssphäre B.

Nachfolgend wird unter Verweis auf die Figuren 2 und 3 die Funktionsweise des Fräskopfes 10 und des Instrumentariums 62 erläutert. Ziel ist eine halbkugelförmige Präparation des Acetabulums 16, so dass eine halbkugelförmige Gelenkschale eines Hüftgelenkes zuverlässig darin verankert werden kann.

Das Acetabulum 16 definiert eine Körperachse 66, die im Normalfall mit der Achse des Oberschenkelhalses zusammenfällt, und es umfasst eine Eintrittsöffnung 68 in die Ausnehmung 12. Durch die Eintrittsöffnung 68 hindurch taucht im Normalfall der Femurkopf in die Ausnehmung 12 ein, und bei der Bearbeitung des Acetabulums 16 der Fräskopf 10. Die Eintrittsöffnung 68 definiert eine Eintrittsebene E. Das Acetabulum 16 ist halbkugelförmig präpariert, wenn die Äquatorialebene A mit der Eintrittsebene E zusammenfällt, der Fräskopf 10 aber nicht tiefer in das Acetabulum 16 eindringen kann.

Grundsätzlich ist es wünschenswert, dass das Acetabulum 16 achsparallel bearbeitet wird. Dies ist dann der Fall, wenn die Körperachse 66 mit der Achse 28 zusammenfällt (Figur 2). Das anfänglich möglicherweise nicht halbkugelförmige Acetabulum 16 wird vom Operateur unter Einwirkung einer distalen Kraft mit dem Fräskopf 10 beaufschlagt, bis dieser in den Knochen eindringt und Knochenmaterial abträgt. Bei achsparalleler Bearbeitung erfolgt das Eindringen des Fräskopfes 10 in axialer Richtung der Achse 28. Anhand der Markierung 54 kann der Operateur erkennen, dass die gewünschte Eintauchtiefe des Fräskopfes 10 ins Acetabulum 16 erreicht ist.

Fluchtet die Markierung 54 mit einem in der Eintrittsebene E liegenden äußeren Rand 70 des Acetabulums 16, kann der Operateur erkennen, dass die Äquatorialebene A und die Eintrittsebene E zusammenfallen und allein die distale Hemisphäre der Bearbeitungssphäre B in das Acetabulum 16 eintaucht. Dies stellt sicher, dass eine halbkugelförmige Präparation des Acetabulums 16 erzielt wird. Anhand der proximalen Schneidelemente 46 bis 50 sowie der weiteren nicht gezeigten proximalen Schneidelemente kann insbesondere auch ein unmittelbar distal bezüglich der Eintrittsebene E gelegener Randbereich 72 des Acetabulums 16 halbkugelförmig präpariert werden. Dies beruht auf der Tatsache, dass sich die Schneidkanten 38 der proximalen Schneidelemente 46 bis 50 distalseitig über die Äquatorialebene A hinaus erstrecken. Am Acetabulum 16 bleibt daher am Randbereich 72 kein sich in radialer Richtung erstreckender wulstförmiger Überstand an Knochenmaterial übrig.

Die weiteren, rein distalen Schneidelemente des Fräskopfes 10 sind derart am Grundkörper 18 angeordnet, dass sie in Rotation die übrige Oberfläche der distalen Hemisphäre der Bearbeitungssphäre B abdecken. Dadurch kann auf einfache Weise insgesamt eine halbkugelförmige Präparation des Acetabulums 16 bei achsparalleler Bearbeitung erzielt werden.

In der Praxis kann es allerdings leicht passieren, beispielsweise aufgrund beschränkter Einsicht in das Operationsgebiet, dass die Achse 28 des Fräskopfes 10 relativ zu Körperachse 66 verkippt ist (Figur 3). Dies resultiert in einer taumelnden Bearbeitung des Acetabulums 16. Auch bei einer taumelnden Bearbeitung kann eine runde, halbkugelförmige Präparation des Acetabulums 16 erzielt werden. Dies ist dadurch begründet, dass der erfindungsgemäße Fräskopf 10 die proximalen Schneidelemente 46 bis 50 aufweist mit den sich in proximaler Richtung über die Äquatorialebene A hinaus erstreckenden Schneidkanten 38.

Bei taumelnder Bearbeitung können diese proximalen Abschnitte der Schneidkanten 38 der proximalen Schneidelemente 46 bis 50 den Randbereich 72 unterhalb der Eintrittsebene E ebenfalls bearbeiten. Wird der Fräskopf 10 relativ zum Acetabulum 16 getaumelt, wobei die Achse 28 beispielsweise um die Körperachse 66 präzediert, kann der Randbereich 72 entlang des kompletten Umfangs der Körperachse 66 bearbeitet werden. Da die Schneidkanten 38 der proximalen Schneidelemente 46 bis 50 ebenfalls auf der Bearbeitungssphäre B liegen (auf der proximalen Hemisphäre), definieren die proximalen Schneidelemente 46 bis 50 zudem Führungselemente bei der taumelnden Bearbeitung. Dies bedeutet, dass bei taumelnder Bearbeitung die Bearbeitungssphäre B sich in der Ausnehmung 12 so abwälzen kann, dass die gewünschte halbkugelförmige Präparation des Acetabulums 16 gelingt. Dies erfolgt, weil sich zugleich mit der Bearbeitung des Knochens 14 die proximalen Schneidelemente 46 bis 50 mittels ihrer Schneidkanten 38 am Knochen 14 abstützen können.

Dass bei taumelnder Bearbeitung tatsächlich eine halbkugelförmige Präparation erzielt wurde, kann der Operateur dann daran erkennen, indem er versucht, den Fräskopf 10 in die achsparallele Konfiguration zu überführen. Auf die vorstehend erläuterte Weise kann er dann anhand der Markierung 54 erkennen, ob die Äquatorialebene A und die Eintrittsebene E bereits zusammenfallen.

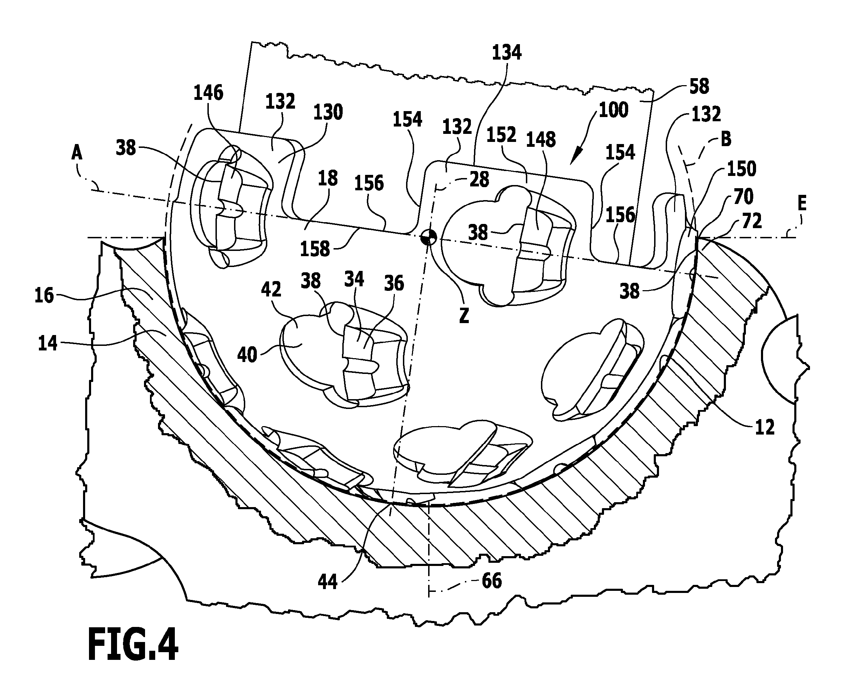

Eine zweite bevorzugte Ausführungsform des erfindungsgemäßen chirurgischen Instrumentes ist in der Figur 4 in einer der Figur 3 entsprechenden Darstellung bei taumelnder Bearbeitung des Acetabulums 16 dargestellt und dort mit dem Bezugszeichen 100 belegt. Der Fräskopf 100 ist ebenfalls Bestandteil des erfindungsgemäßen Instrumentariums 62, und über eine in der Zeichnung nicht dargestellte Verbindungseinrichtung kann er ebenfalls lösbar mit dem Schaft 58 verbunden werden.

Der Fräskopf 100 ist weitgehend identisch aufgebaut wie der Fräskopf 10. Für gleiche und gleichwirkende Merkmale der Fräsköpfe 10 und 100 werden daher dieselben Bezugszeichen benutzt. Die mit dem Fräskopf 10 erzielbaren Vorteile können mit dem Fräskopf 100 ebenfalls erzielt werden.

Vom Fräskopf 10 unterscheidet sich der Fräskopf 100 durch die Art und Weise, wie dessen Randbereich 130 gebildet ist. Der Randbereich 130 folgt ebenfalls der sphärischen Kontur des Grundkörpers 18 und steht in proximaler Richtung über die Äquatorialebene A hinaus. Der Randbereich 130 reicht ungefähr bis zum 18. Breitengrad der vom Grundkörper 18 definierten Sphäre in proximaler Richtung.

Der Randbereich 130 ist in Umfangsrichtung der Achse 28 in eine Mehrzahl von Randsegmenten 132 unterteilt, und zwar fünf Stück, wovon in der Zeichnung nur drei Randsegmente 132 zu erkennen sind. Die Randsegmente 132 sind identisch ausgebildet, insbesondere in Form von vom Grundkörper 18 im Übrigen hervorstehenden Rechteckzinnen. In Umfangsrichtung der Achse 28 überdecken sie jeweils ungefähr einen Winkelbereich von 30 Längengraden. Ferner sind sie in Umfangsrichtung der Achse 28 paarweise voneinander beabstandet, und zwar um unterschiedliche Winkel.

Im Bereich jedes Randsegmentes 132 ist ein Schneidelement 36 gebildet. Vorliegend handelt es sich dabei um proximale Schneidelemente 146, 148 und 150, welche zugleich distale Schneidelemente sind. Dies bedeutet, dass sich die Schneidkanten 38 der Schneidelemente 146 bis 150 über die Äquatorialebene A sowohl in proximaler als auch in axialer Richtung erstrecken. Bezüglich der Äquatorialebene A sind die Schneidelemente 146 bis 150 symmetrisch am Grundkörper 18 angeordnet, wobei sich deren Schneidkanten 38 proximalseitig und distalseitig jeweils ungefähr bis zum 10. Breitengrad erstrecken. Proximal steht ein durch die Ausgestaltung der Randsegmente 132 unterbrochener freier Rand 134 des Randbereiches 130 also über die Schneidkanten 38 der Schneidelemente 146 bis 150 hinaus. Wie im Fall des Fräskopfes 10 verringert dies im Fall des Fräskopfes 100 die Gefahr einer Verletzung des Operateurs und des Patienten. Zwischen den proximalen Enden der Schneidkanten 38 der Schneidelemente 146 bis 150 und dem freien Rand 134 sind dementsprechend Zwischenabschnitte 152 an den Randsegmenten 132 gebildet.

Die Randsegmente 132 sind so bemessen, dass innerhalb der von ihnen überdeckten Längengradbereichen von ungefähr 30 Längengraden auch die proximale Hälfte der jeweiligen Durchbrechungen 42 zur Abfuhr von Knochenmaterial in den Randsegmenten 132 gebildet ist.

Zwischen je zwei Randsegmenten 132 ist jeweils eine Ausnehmung 154 gebildet. Die Ausnehmungen 154 umfassen jeweils einen Boden 156, der in der Äquatorialebene A liegt. Die Böden 156 bilden dadurch gemeinsam eine Markierung 158 am Äquator 26, die denselben Zweck wie die vorstehend genannte Markierung 54 erfüllt.

Figur 4 zeigt den Fräskopf 100 bei taumelnder Bearbeitung des Acetabulums 16, bei der die Schneidkanten 38 der proximalen Schneidelemente 146 zum Einen Führungsglieder zum Abwälzen der Bearbeitungssphäre B in der Ausnehmung 12 bilden und zum Anderen ermöglichen, Knochenmaterial am Randbereich 72 des Acetabulums 16 distalseitig der Eintrittsebene E abzutragen. Anders als der Fräskopf 10 kann der Fräskopf 100 aufgrund der Anordnung der Schneidelemente 146 bis 150 am Grundkörper 18 auch dann noch zuverlässig zur taumelnden Bearbeitung eingesetzt werden, wenn die Achse 28 bezüglich der Körperachse 66 um einen Winkel von ungefähr 10° verkippt ist. Allerdings wird zum Einbringen des Fräskopfes 100 in den Körper u. U. ein etwas größerer Zugang erforderlich sein als für den Fräskopf 10, gleichen Durchmesser der jeweiligen Grundkörper 18 vorausgesetzt.

Auch bei achsparalleler Bearbeitung des Acetabulums 16 mit dem Fräskopf 100 (nicht gezeigt) kann zuverlässig eine halbkugelförmige Präparation des Acetabulums 16 vorgenommen werden, da insbesondere der Randbereich 72 mit den distalen Abschnitten der proximalen Schneidelemente 146 bis 150 präpariert werden kann.

Wie im Fall des Fräskopfes 10 überdecken die weiteren, distalen Schneidelemente 36 des Fräskopfes 100 die übrige Oberfläche der distalen Hemisphäre der Bearbeitungssphäre B. Daher kann auch beim Fräskopf 100 sowohl achsparallel als auch taumelnd eine halbkugelförmige Ausnehmung 12 präpariert werden. Anhand der Markierung 158 kann entsprechend der vorstehend erläuterten Weise vom Operateur erkannt werden, dass die Äquatorialebene A und die Eintrittsebene E zusammenfallen und das Präparationsergebnis erreicht ist.

## Patentansprüche

1. Chirurgisches Instrument zur Erzeugung einer halbkugelförmigen Ausnehmung (12) in einem Knochen (14), das um eine Drehachse (28) drehend antreibbar ist und mindestens ein distales Schneidelement (36) mit einer Schneidkante (38) zur Abtragung von Knochenmaterial umfasst, wobei die Schneidkante (38) in Rotation des Instrumentes (10; 100) um die Drehachse (28) eine Bearbeitungssphäre (B) mit einer in die Ausnehmung (12) einführbaren distalen Hemisphäre, einem Äquator und einer proximalen Hemisphäre definiert, wobei das Instrument (10; 100) einen als Hohlkörper ausgebildeten Grundkörper (18) umfasst, an dem die Schneidelemente (36, 46, 48, 50; 146, 148, 150) festgelegt sind, und wobei das Instrument (10; 100) mehrere proximale Schneidelemente (46, 48, 50; 146, 148, 150) mit einer jeweiligen Schneidkante (38) umfasst, die sich ausgehend von der Äquatorialebene (A) der Bearbeitungssphäre (B) oder im Wesentlichen von der Äquatorialebene (A) in proximaler Richtung erstreckt, wobei die Schneidkanten (38) der proximalen Schneidelemente (46, 48, 50; 146, 148, 150) in Umfangsrichtung der Drehachse (28) paarweise voneinander beabstandet sind, **dadurch gekennzeichnet, dass** die Schneidkanten (38) der proximalen Schneidelemente (46, 48, 50; 146, 148, 150) in Umfangsrichtung der Drehachse (28) ungleichmäßig voneinander beabstandet sind.

2. Instrument nach Anspruch 1, **dadurch gekennzeichnet, dass** sich die Schneidkante (38) mindestens eines proximalen Schneidelementes (46, 48, 50; 146, 148, 150) auf der proximalen Hemisphäre erstreckt.

3. Instrument nach einem der voranstehenden Ansprüche, **dadurch gekennzeichnet, dass** sich die Schneidkante (38) mindestens eines proximalen Schneidelementes (46, 48, 50; 146, 148, 150) über die Äquatorialebene (A) der Bearbeitungssphäre (B) hinaus in distaler Richtung erstreckt.

4. Instrument nach Anspruch 3, **dadurch gekennzeichnet, dass** mindestens ein proximales Schneidelement (146, 148, 150) symmetrisch bezüglich der Äquatorialebene (A) der Bearbeitungssphäre (B) am Instrument (10; 100) angeordnet ist.

5. Instrument nach einem der voranstehenden Ansprüche, **dadurch gekennzeichnet, dass** die proximalen Schneidelemente (46, 48, 50; 146, 148, 150) identisch ausgebildet sind.

6. Instrument nach einem der voranstehenden Ansprüche, **dadurch gekennzeichnet, dass** sich die Schneidkante (38) mindestens eines distalen Schneidelementes (36) in proximaler Richtung bis zur Äquatorialebene (A) der Bearbeitungssphäre (B) oder im Wesentlichen bis zur Äquatorialebene (A) erstreckt.

7. Instrument nach Anspruch 6, **dadurch gekennzeichnet, dass** sich die Schneidkante (38) des Schneidelementes (36) in proximaler Richtung über die Äquatorialebene (A) hinaus erstreckt.

8. Instrument nach einem der voranstehenden Ansprüche, **dadurch gekennzeichnet, dass** die Schneidkanten (38) der Schneidelemente (36, 46, 48, 50; 146, 148, 150) des Instrumentes (10; 100) jeweils längs eines Meridians der Bearbeitungssphäre (B) verlaufen.

9. Instrument nach einem der voranstehenden Ansprüche, **dadurch gekennzeichnet, dass** der Grundkörper (18) einen über die Äquatorialebene (A) der Bearbeitungssphäre (B) in proximaler Richtung hinaus ragenden Randbereich (30; 130) umfasst.

10. Instrument nach Anspruch 9, **dadurch gekennzeichnet, dass** der Randbereich (30; 130) in proximaler Richtung über das proximale Ende der Schneidkante (38) mindestens eines proximalen Schneidelementes (46, 48, 50; 146, 148, 150) hinaus ragt.

11. Instrument nach Anspruch 9 oder 10, **dadurch gekennzeichnet, dass** der Randbereich (30) in Umfangsrichtung der Drehachse (28) zusammenhängend ausgebildet ist.

12. Instrument nach Anspruch 11, **dadurch gekennzeichnet, dass** der Randbereich (30) in Umfangsrichtung der Drehachse (28) einstückig ausgebildet ist.

13. Instrument nach Anspruch 9 oder 10, **dadurch gekennzeichnet, dass** der Randbereich (130) in Umfangsrichtung der Drehachse (28) in zwei oder mehr voneinander beabstandete Randsegmente (132) unterteilt ist, so dass zwischen je zwei Randsegmenten (132) Ausnehmungen (154) angeordnet sind.

14. Instrument nach einem der voranstehenden Ansprüche, **dadurch gekennzeichnet, dass** der Grundkörper (18) eine Markierung (54; 158) umfasst, anhand derer der Äquator der Bearbeitungssphäre (B) erkennbar ist.

15. Chirurgisches Instrumentarium, umfassend mindestens ein Instrument (10; 100) nach einem der vorstehenden Ansprüche sowie eine Antriebseinrichtung (60), wobei das mindestens eine Instrument (10; 100) lösbar mit der Antriebseinrichtung (60) verbindbar und von dieser drehend antreibbar ist.

## Claims

1. Surgical instrument for creating a hemispherical recess (12) in a bone (14), which is rotatingly drivable about a rotation axis (28) and comprises at least one distal cutting element (36) with a cutting edge (38) for removing bone material, wherein the cutting edge (38) defines, during the rotation of the instrument (10; 100) about the rotation axis (28), a shaping sphere (B) with a distal hemisphere which can be introduced into the recess (12), an equator and a proximal hemisphere, wherein the instrument (10; 100) comprises a base body (18) configured as a hollow body on which the cutting elements (36, 46, 48, 50; 146, 148, 150) are fixed, and wherein the instrument (10; 100) comprises a plurality of proximal cutting elements (46, 48, 50; 146, 148, 150) with a respective cutting edge (38) which extends, starting from the equatorial plane (A) of the shaping sphere (B) or substantially from the equatorial plane (A) in the proximal direction, wherein the cutting edges (38) of the proximal cutting elements (46, 48, 50; 146, 148, 150) are spaced apart from one another in pairs in the circumferential direction of the rotation axis (28), **characterised in that** the cutting edges (38) of the proximal cutting elements (46, 48, 50; 146, 148, 150) are spaced apart from one another unevenly in the circumferential direction of the rotation axis (28).

2. Instrument according to claim 1, **characterised in that** the cutting edge (38) of at least one proximal cutting element (46, 48, 50; 146, 148, 150) extends on the proximal hemisphere.

3. Instrument according to one of the preceding claims, **characterised in that** the cutting edge (38) of at least one proximal cutting element (46, 48, 50; 146, 148, 150) extends in the distal direction beyond the equatorial plane (A) of the shaping sphere (B).

4. Instrument according to claim 3, **characterised in that** at least one proximal cutting element (146, 148, 150) is arranged symmetrically on the instrument (10; 100) in relation to the equatorial plane (A) of the shaping sphere (B).

5. Instrument according to one of the preceding claims, **characterised in that** the proximal cutting elements (46, 48, 50; 146, 148, 150) are identically formed.

6. Instrument according to one of the preceding claims, **characterised in that** the cutting edge (38) of at least one distal cutting element (36) extends in the proximal direction as far as the equatorial plane (A) of the shaping sphere (B) or substantially as far as the equatorial plane (A).

7. Instrument according to claim 6, **characterised in that** the cutting edge (38) of the cutting element (36) extends beyond the equatorial plane (A) in the proximal direction.

8. Instrument according to one of the preceding claims, **characterised in that** the cutting edges (38) of the cutting elements (36, 46, 48, 50; 146, 148, 150) of the instrument (10; 100) each extend along a meridian of the shaping sphere (B).

9. Instrument according to one of the preceding claims, **characterised in that** the base body (18) comprises an edge region (30; 130) extending beyond the equatorial plane (A) of the shaping sphere (B) in the proximal direction.

10. Instrument according to claim 9, **characterised in that** the edge region (30; 130) extends in the proximal direction beyond the proximal end of the cutting edge (38) of at least one proximal cutting element (46, 48, 50; 146, 148, 150).

11. Instrument according to claim 9 or 10, **characterised in that** the edge region (30) is formed to be continuous in the circumferential direction of the rotation axis (28).

12. Instrument according to claim 11, **characterised in that** the edge region (30) is formed in one piece in the circumferential direction of the rotation axis (28).

13. Instrument according to claim 9 or 10, **characterised in that** the edge region (130) is subdivided in the circumferential direction of the rotation axis (28) into two or more edge segments (132) spaced apart from one another so that recesses (154) are arranged between each pair of edge segments (132).

14. Instrument according to one of the preceding claims, **characterised in that** the base body (18) comprises a marking (54; 158), by means of which the equator of the shaping sphere (B) is recognisable.

15. Surgical instrumentation comprising at least one instrument (10; 100) according to one of the preceding claims and a drive device (60), wherein the at least one instrument (10; 100) is releasably connectable to the drive device (60) and is rotatingly drivable thereby.

## Revendications

1. Instrument chirurgical pour la réalisation d'un évidement (12) de forme semi-sphérique dans un os (14), qui peut être entraîné en rotation autour d'un axe de rotation (28) et comprend au moins un élément de coupe distal (36) avec une arête de coupe (38) pour l'enlèvement de matière osseuse, instrument dans lequel l'arête de coupe (38) définit, lorsque l'instrument (10; 100) est en rotation autour de l'axe de rotation (28), une sphère d'usinage (B) présentant une hémisphère distale pouvant être introduite dans l'évidement (12), un équateur et une hémisphère proximale,
dans lequel l'instrument (10; 100) comporte un corps de base (18) réalisé sous forme de corps creux, sur lequel sont fixés les éléments de coupe (36, 46, 48, 50; 146, 148, 150),
et dans lequel l'instrument (10; 100) comporte plusieurs éléments de coupe proximaux (46, 48, 50; 146, 148, 150) avec une arête de coupe (38) respective, qui s'étend à partir du plan équatorial (A) de la sphère d'usinage (B) ou sensiblement à partir du plan équatorial (A) en direction proximale, les arêtes de coupe (38) des éléments de coupe proximaux (46, 48, 50; 146, 148, 150) étant espacées mutuellement les unes des autres par paires, dans la direction périphérique de l'axe de rotation (28),
**caractérisé en ce que** les arêtes de coupe (38) des éléments de coupe proximaux (46, 48, 50; 146, 148, 150) sont espacées de manière irrégulière les unes des autres dans la direction périphérique de l'axe de rotation (28) .

2. Instrument selon la revendication 1, **caractérisé en ce que** l'arête de coupe (38) d'au moins un élément de coupe proximal (46, 48, 50; 146, 148, 150) s'étend sur l'hémisphère proximale.

3. Instrument selon l'une des revendications précédentes, **caractérisé en ce que** l'arête de coupe (38) d'au moins un élément de coupe proximal (46, 48, 50; 146, 148, 150) s'étend au-delà du plan équatorial (A) de la sphère d'usinage (B), en direction distale.

4. Instrument selon la revendication 3, **caractérisé en ce qu'**au moins un élément de coupe proximal (146, 148, 150) est agencé symétriquement par rapport au plan équatorial (A) de la sphère d'usinage (B) sur l'instrument (10; 100).

5. Instrument selon l'une des revendications précédentes, **caractérisé en ce que** les éléments de coupe proximaux (46, 48, 50; 146, 148, 150) sont de configuration identique.

6. Instrument selon l'une des revendications précédentes, **caractérisé en ce que** l'arête de coupe (38) d'au moins un élément de coupe distal (36) s'étend en direction proximale, jusqu'au plan équatorial (A) de la sphère d'usinage (B) ou sensiblement jusqu'au plan équatorial (A).

7. Instrument selon la revendication 6, **caractérisé en ce que** l'arête de coupe (38) de l'élément de coupe (36) s'étend en direction proximale, au-delà du plan équatorial (A).

8. Instrument selon l'une des revendications précédentes, **caractérisé en ce que** les arêtes de coupe (38) des éléments de coupe (36, 46, 48, 50; 146, 148, 150) de l'instrument (10; 100) s'étendent chacune respectivement le long d'un méridien de la sphère d'usinage (B).

9. Instrument selon l'une des revendications précédentes, **caractérisé en ce que** le corps de base (18) comprend une zone de bordure (30; 130), qui, en direction proximale, s'étend au-delà du plan équatorial (A) de la sphère d'usinage (B).

10. Instrument selon la revendication 9, **caractérisé en ce que** la zone de bordure (30; 130) s'étend, en direction proximale, au-delà de l'extrémité proximale de l'arête de coupe (38) d'au moins un élément de coupe proximal (46, 48, 50; 146, 148, 150).

11. Instrument selon la revendication 9 ou la revendication 10, **caractérisé en ce que** la zone de bordure (30) est réalisée continue dans la direction périphérique de l'axe de rotation (28).

12. Instrument selon la revendication 11, **caractérisé en ce que** la zone de bordure (30) est réalisée d'un seul tenant dans la direction périphérique de l'axe de rotation (28).

13. Instrument selon la revendication 9 ou la revendication 10, **caractérisé en ce que** la zone de bordure (130) est subdivisée, dans la direction périphérique de l'axe de rotation (28), en deux segments de bordure (132) ou davantage, qui sont espacés mutuellement, de sorte que des évidements (154) sont agencés respectivement entre à chaque fois deux segments de bordure (132).

14. Instrument selon l'une des revendications précédentes, **caractérisé en ce que** le corps de base (18) comporte un marquage (54; 158) à l'aide duquel il est possible d'identifier l'équateur de la sphère d'usinage (B).

15. Instrumentation chirurgicale comprenant au moins un instrument (10; 100) selon l'une des revendications précédentes, ainsi qu'un dispositif d'entraînement (60), ledit au moins un instrument (10; 100) pouvant être relié de manière amovible au dispositif d'entraînement (60) et être entraîné en rotation par celui-ci.
